# EUROPEAN PATENT SPECIFICATION

(11) **EP 3 222 314 B1**
(45) Date of publication and mention of the grant of the patent: **21.11.2018**
(21) Application number: 17162081.8
(22) Date of filing: 21.03.2017
(51) Int. Cl.: A61M 15/08

(54) **NASAL SPRAY APPARATUS**
NASENSPRAYVORRICHTUNG
APPAREIL DE PULVÉRISATION NASALE

(30) Priority: 21.03.2016 US 201662310824 P
(43) Date of publication of application: 27.09.2017
(73) Proprietor: Feldman, Joseph, 6578929 Tel Aviv (IL); Primor, Nitsan, 64237 Tel Aviv (IL)
(72) Inventor: Feldman, Joseph, 6578929 Tel Aviv (IL); Primor, Nitsan, 64237 Tel Aviv (IL)
(74) Representative: Modiano, Gabriella Diana

(56) References cited:
- WO-A1-2011/067752
- WO-A2-2007/127894
- WO-A2-2011/153406
- DE-A1-102012 200 545
- GB-A- 2 443 318
- US-A- 5 292 346
- US-A1- 2015 266 042

## Description

### FIELD OF THE INVENTION

The present invention relates to nasal spray apparatus.

### BACKGROUND OF THE INVENTION

The present invention concerns devices for administrating medicated (or non-medicated) fluid substances, in the form of droplets or aerosol spray, to the nostrils of patients suffering from nasal congestion, typically due to allergic diseases ("rhinitis").
Clinical symptoms of rhinitis, such as a runny nose, an itchy nose, post nasal drainage of mucus and, in extreme cases, congested secondary air passages, may be attributed to various etiologies. The common etiologies are viral infection, such as infectious rhino sinusitis. Other etiologies include allergic, perennial, or seasonal rhinitis, also known as "hay fever", non-allergic vasomotor rhinitis, eosinophyllic rhinitis and nasal polyps.

Existing methods of treatment of the above mentioned rhinitis symptoms include systematic use of medications, such as antihistamines and decongestants, or local treatment with steroid spray, D.S.C.G. or local decongestants. There have been also attempts to treat rhinitis locally by applying a "fog stream", i.e. a stream of water at a temperature of approximately 42° C. These medications may be taken orally or may be administered directly to the tissue of the nostrils by means of a nasal spray.

The majority of the commercially available atomizers or aerosol spray devices comprise a pressurized propellant gas vessel containing the active substance in a liquid form (e.g. antihistamine, sea water), with a suitable shaped cap so that by pressing thereon, a valve is opened and the spray is ejected into the user's nostril for as long as the cover is compressed. Other models have a pumping mechanism rather than compressed gas. According to these models each time the cover is pressed a pre-selected amount of the substance is dispensed.

It is appreciated that none of the existing treatments described above, nor any combinations thereof, completely relieve rhinitis related symptoms. Therefore, a large population is helplessly exposed to the irritating discomforts of rhinitis symptoms.

As an alternative means of treatment of rhinitis, a line of nasal congestion treatment devices have been developed based on nostril illumination of the type disclosed in US Patent No. 5,683,436 (Nov.4, 1997 - Mendes, et al.). This patent provides a method and apparatus for therapeutic illumination which are particularly suited for treatment of rhinitis and for treatment of various nasal conditions. As therein described, the operation of these biostimulative illumination apparatus includes the use of Light Emitting Diodes (LED) configured to radiate non-coherent red light at a preferred wavelength of 660 nm into the nostril.

Document DE102012200545 discloses a dispenser for discharging media, in particular liquids, with a base unit and a discharge head with discharge. The discharge head has a first part section which has a fastening device by means of which it is fixedly attached to the media store on the base unit and which supports a second section movably formed relative to the first section and having an actuating handle connected to a coupling such that a displacement of the actuating handle relative to the first section results in a displacement of the coupling with respect to the first section.

WO2011067752 discloses a device for the treatment of rhinitis by biostimulative illumination. The device comprises a pair of LEDs containing probes adapted to be inserted into the nostrils of a patient. The probes are normally flexibly attracted one against the other. The probe casing accommodates an electric power source, an ON/OFF normally open micro-switch and circuit means for activating/deactivating the LEDs . The casing is made of at least partly elastomeric material whereby the micro-switch becomes activated by pressing against a side-wing of the casing also causing the probes to spread apart from each other and the micro-switch remains closed for as long as the probes are kept apart from each other.

Document WO2007127894 discloses devices, kits, systems and methods that can be used to deliver and activate a photosensitizing composition in a cavity. A device includes a member having a base portion, an insert portion adapted for insertion into the cavity, and a pocket adapted for communication with a waveguide which is connected to a light source for delivering light to the device. The pocket includes a light dispersing section that is adapted for light communication with distal end of the waveguide and desired illumination pattern for photodisinfection of the cavity is provided by at least one of the elements selected from the group consisting of: surface finish of the light dispersing section, geometry of the light dispersing section, surface finish of the member, geometry of the member, and a combination thereof.

Accordingly, there is a long felt need for a spraying device that is capable of administering the medicated (or non-medicated) fluid while illuminating the area that is being sprayed, and it would be desirable to have a spraying device that permits the performing of these two operations by a single action of the user.

### SUMMARY OF THE INVENTION

The present invention combines the two therapeutic methodologies into a unified, self-contained nasal spray apparatus. The single nasal spray apparatus activates an LED to provide biostimulative illumination and, simultaneously, administers the active fluid. According to embodiments of the invention, both operations are initiated by depressing the nozzle cap.

There is provided according to the present invention a cap for a nasal spray apparatus, the cap including a spray dispensing mechanism and further including a base sealingly mountable on a container of liquid substance to be dispensed; an intermediate section arranged for reciprocating movement relative to the base the intermediate section including the spray dispensing mechanism and having a spray dispensing passage; a cover-section having a depressible flexible portion, coupled to the intermediate-section; and a Light Emitting Diode (LED) connected to a power source through an electric circuit, the LED mounted adjacent the spray dispensing passage so as to be inserted into a nostril therewith; wherein the depressible flexible portion is arranged and configured to cause closure of the electric circuit for activating the LED and to actuate the spray dispensing mechanism, to provide both illumination and spray of liquid substance into the nostril.

There is provided according to a general aspect of the invention an atomizer apparatus for treating nasal congestion and the like conditions of a patient. The apparatus comprises a vessel containing a therapeutic fluid intended to be administered in droplet (spray) form into a nostril of the patient. According to some embodiments of the invention, valve means are provided for controlling the delivery of the fluid by a push-button nozzle cap member mounted on the vessel. By pressing the cap member, communication is established between the valve means and spray dispensing passage in the cap configured to be inserted into the nostril for moistening the nostril by the fluid. A Light Emitting Diode (LED) is provided, adjacent the spray dispensing passage, so as to be inserted into the nostril therewith. Further provided are an electric power source connectable via switching means to the LED and means for selectively actuating the switching means by the patient.

### BRIEF DESCRIPTION OF THE DRAWINGS

The objects, constructional features and advantages of the present invention will be more readily understood in the light of the ensuing description of a preferred embodiment thereof, given by way of example only, with reference to the accompanying drawings, wherein:-
Fig. 1 is a general side-view of nasal spray apparatus constructed and operative according to preferred embodiments of the invention;
Fig. 2 is a perspective exploded view of a cap for the nasal spray apparatus of Fig. 1;
Fig. 3A is a side-view of nozzle cap member of Fig. 2;
Fig. 3B is a cross-section taken along A--A of Fig. 3A;
Fig. 3C is a cross-section taken along B--B of Fig. 3A;
Fig. 4A is a top-view of the nozzle cap member of Fig. 3A;
Fig. 4B is a cross-section taken along C--C of Fig. 4A; and,
Fig. 4C is a cross-section taken along D--D of Fig. 4A.

### DETAILED DESCRIPTION OF THE INVENTION

The present invention relates to a cap for a nasal spraying device that is capable of administering a medicated (or non-medicated) fluid, in spray form, while illuminating the area that is being sprayed. In particular, the nasal spraying device permits performing these two operations substantially simultaneously, or one immediately after the other, by a single action of the user with a single device. In this way, more effective treatment for rhinitis and the like can be provided by a single device in a single operation.

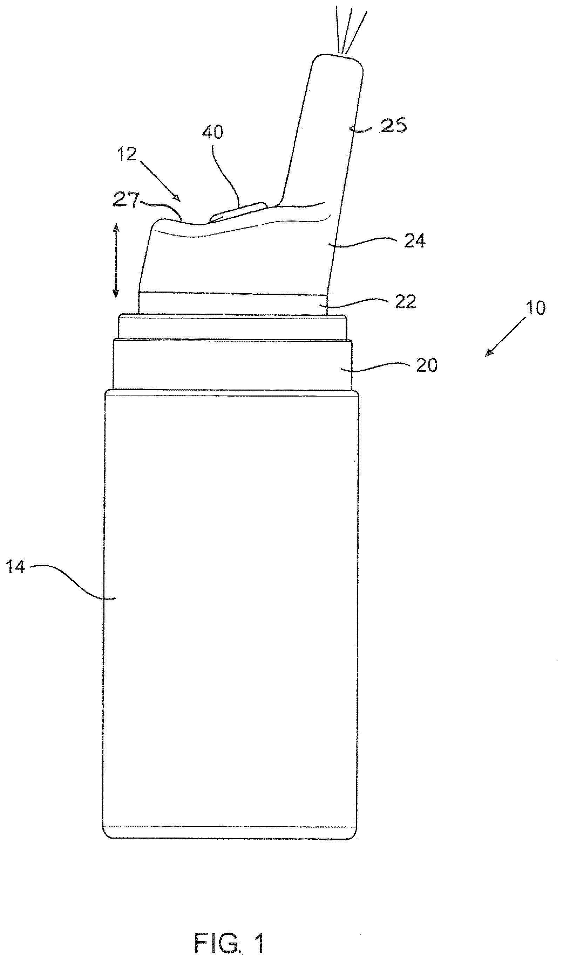

Fig. 1 illustrates a nasal spray apparatus according to a preferred embodiment of the present invention, generally designated **10**. The embodiment of nasal spray apparatus **10** utilizes an aerosol delivery mechanism. A nozzle cap member **12**, in its assembled state, is sealingly mounted onto a container **14**, which can hold a propellant gas as well as a liquid substance for spraying. Typically in aerosol mechanisms, a long plastic tube runs from the bottom of the container up to a valve system (not shown) at the top of the container. The valve can have a narrow channel running from an inlet near the bottom of a head piece to a small nozzle at the top. A spring pushes the head piece up, so the channel inlet is blocked by a tight seal. When the head piece is pushed down, the inlet slides below the seal, opening a passage from the inside of the container to the spray passage. The high-pressure propellant gas drives the liquid product up the plastic tube, through the spray passage and out through a nozzle or spray orifice. The narrow nozzle serves to atomize the flowing liquid, breaking it up into tiny drops, which form a fine spray.

In this embodiment of the invention, depressing the cap member towards the container will cause dispensing of liquid from the container and into the nostril of the user. A flexible portion, possibly including a depressible actuator button **40**, is provided in cap member **12**, the construction and function of which will be described in greater detail below. Essentially, pressing down on the flexible portion opens the valve and allows pressurized contents from the container to be sprayed out through the spray orifice. The illumination effect is improved when the spray drops are relatively small in size.

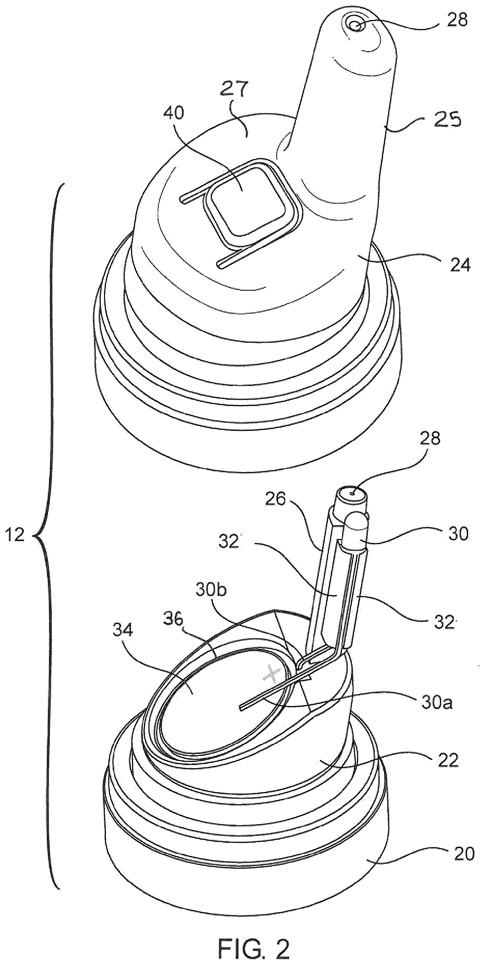

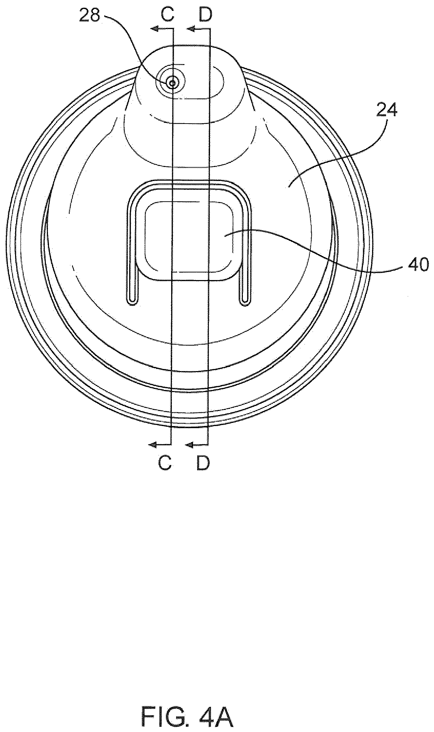

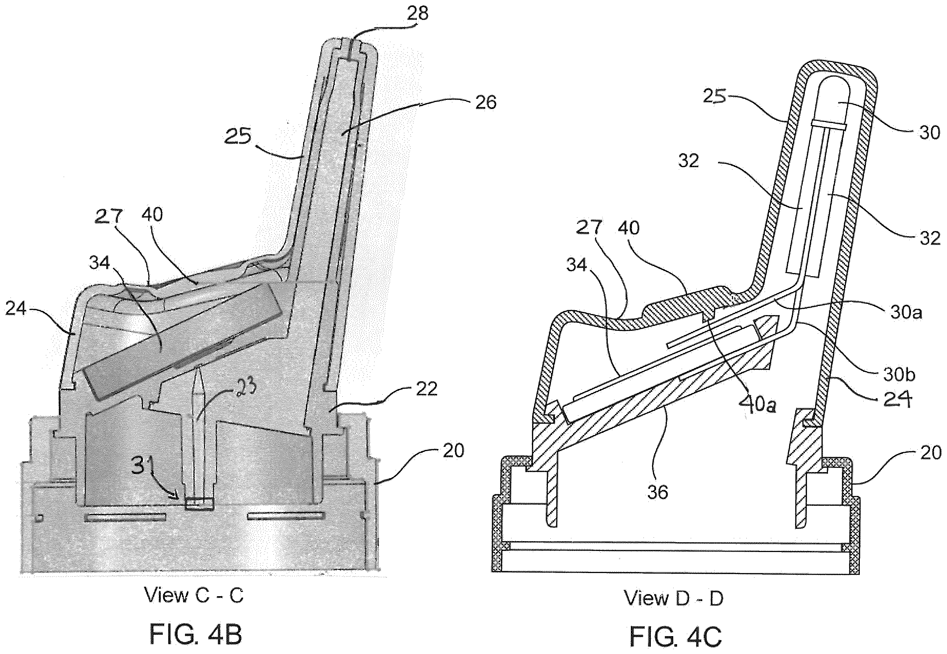

With further reference to Figs. 2, 4B and 4C, the cap member **12** according to the illustrated embodiment is composed of three assembled-together sections: A base-section **20**, which is coupled to the container **14**; an intermediate-section **22** including a spray dispensing mechanism, the intermediate section configured and adapted for reciprocating movement (to slide up and down) relative to the base-section to operate a gas/fluid valve, generally indicated **23**, of the container by any known means (not shown); and a cover-section **24**, including a dispensing nozzle **25**, press-fitted (or otherwise assembled) to the intermediate-section **22**. The spring in the aerosol valve causes intermediate-section **22** to return to its usual position.

In more detail, the intermediate-section **22** is formed with a tubular spray passage **26** which is seated inside dispensing nozzle **25**, and which has a dispensing orifice **28**. When the apparatus is operated, the spray liquid passes from the container, through the spray passage **26** and is dispensed from orifice **28**. The intermediate-section **22** further accommodates a Light Emitting Diode (LED) **30** mounted adjacent spray passage **26**, preferably juxtaposed to the spray orifice **28**. Preferably, LED **30** emits non-coherent light radiation having a narrow bandwidth centered at a wavelength suitable for rhinitis treatment, typically 660nm.

Preferably, the liquid to be dispensed includes a photosensitizer, such as methylene blue. The photosensitizer increases the sensitivity of bacteria and other organisms or substances in the nostril to electromagnetic radiation, especially visible light, increasing the absorption of light into their cells and disrupting their biochemical balance. In this way, the effect of the light radiation is multiplied and substantially improves the effect of the treatment.

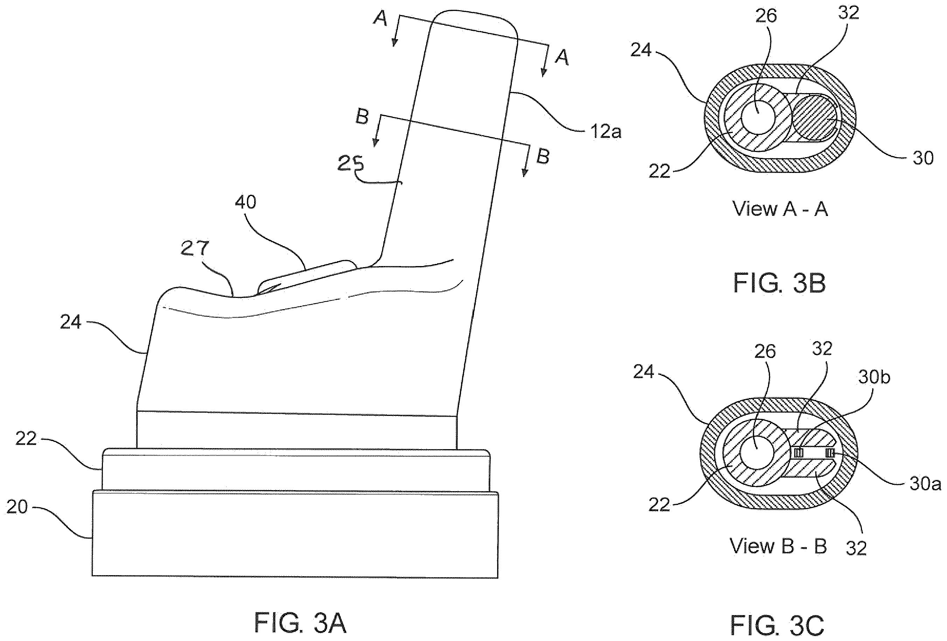

The LED is mounted adjacent the spray dispensing passage so as to be inserted into a nostril together therewith. LED **30** is mounted in a support **32** formed of insulating material including two channels or bores which holds and separates the terminals, anode **30a** and cathode **30b**, of the LED. This structure can be seen most clearly in Figs. 3A, 3B and 3C. As can be seen in Fig. 3C, the tubular spray passage **26** is affixed to or integrally formed with the support **32**, through which pass terminals **30a** and **30b** of the LED. In this embodiment, as shown in Fig. 3B, the LED **30** is juxtaposed with the upper end of spray passage **26**, preferably adjacent the spray orifice **28**. LED **30** is connected to a power source through an electric circuit, and the depressible flexible portion is arranged and configured to cause the electric circuit to close to activate the LED and also to actuate the spray dispensing mechanism, to provide both illumination and spray of liquid substance into the nozzle. Preferably, the LED illuminates the spray as well as the nostril.

A power source, here shown as a coin-type battery **34**, is seated within a battery compartment **36**. LED cathode **30b** is disposed in battery compartment **36** under battery **34** and contacts the bottom side of the battery **34**, the negative pole of the battery, while the anode **30a** is disposed above - but is flexibly kept a small distance away from - the upper side of the battery, the positive pole of the battery. It will be appreciated that battery **34** can be fitted in battery compartment **36** upside down, i.e. its negative pole is facing upward and positive pole is facing downward while the terminals of LED **30** are switched wherein anode **30a** is disposed in battery compartment **36** under battery **34** and makes contact with the positive pole of the battery **34** and cathode **30b** is displace above the battery a small distance away from it.

The cover-section **24** is placed over the intermediate-section **22**. It comprises a flexible upper wall **27** which can be used to actuate the spray apparatus. Upper wall **27** is sufficiently flexible to be depressed and engage a terminal of the LED so as to cause the terminal to contact the battery and light up the LED. Preferably, upper wall **27** can continue to press on the intermediate section **22** until it slides relative to base **20** and actuates the aerosol dispenser.

According to some embodiments of the invention, a push-button **40** is provided protruding from the upper wall **27** for ease of actuation. A further protrusion **40a** (best seen in Figs. 4B and 4C) can be formed at the bottom side of button **40** disposed so that by pressing the button down, protrusion 40a depresses LED anode **30a** until it contacts the upper side of the battery **34**, closing the electric circuit which includes the battery and the LED, and lighting the LED. It will be appreciated that push-button **40** can be integrally formed with upper wall **27** of section **24** and/or protrusion **40a** can be integrally formed with upper wall **27**. Continued pressing of push-button **40** causes intermediate section **22** to slide relative to base **20**, causing a valve actuator **23** to open the valve **31**, as in conventional aerosol mechanisms.

In the light of the foregoing description it will be clearly understood that the operation of the apparatus according to the present invention may be carried out in either manner: By merely pressing the button **40** - the LED will become activated and a biostimulative treatment can be achieved for as long as recommended; in addition, by further, more forceful pressing of button **40** section **24** will move section **22** downward and spraying of liquid from the container **14** will take place as in the conventional nasal treatment devices, while LED **30** is activated according to the present invention. In this way, both therapeutic treatments can be provided at the same time to the nostril of a user.

Those skilled in the art to which this invention pertains will readily appreciate that numerous changes, variations and modifications can be effectuated without departing from the true spirit and scope of the invention as defined in and by the appended claims. For example, a manually operated switch can be provided for closing the circuit and lighting the LED, instead of the push-button described above. The switch can be connected on one side to one of the terminals of the LED and the compatible pole of the battery, which can be disposed anywhere in the cap. The other terminal of the LED is connected to the other pole of the battery. For operation, the user actuates the switch which closes the circuit and lights the LED. Thereafter, the user can continue to press the switch mounted on the flexible intermediate portion and the pressure would open the valve of the aerosol and release the spray.

Instead of aerosol, the present invention alternatively can utilize any known mechanism of spray delivery, such as an atomizer operating under the Venturi effect, or a nasal spray pump mechanism. In the latter case, pressing down on the push-button or flexible intermediate portion would cause contact of the terminals with the battery to light the LED and continued pressure would produce a spray of substance out of nozzle by means of a positive displacement pump that acts directly on the fluid. As known, the pump draws liquid up a siphon tube from the bottom of the container and forces it through the nozzle or spray orifice.

In the case of a mechanism for creating spray other than the aerosol described above, the cap for the spray apparatus would also include three assembled-together sections as described above: A base-section **20**, which is sealingly coupled to the liquid container; an intermediate-section **22** including a spray dispensing mechanism, the intermediate section configured and adapted for reciprocating movement (to slide up and down) relative to the base-section to release liquid from the container; and a cover-section **24**, including a dispensing nozzle **25**, press-fitted (or otherwise assembled) to the intermediate-section.

In more detail, the intermediate-section **22** is formed with a tubular spray passage **26** which is seated inside dispensing nozzle **25**, and which has a dispensing orifice **28**. When the apparatus is operated, the spray liquid passes from the container, through the spray passage **26** and is dispensed from orifice **28**. The intermediate-section **22** further accommodates a Light Emitting Diode (LED) **30** mounted adjacent spray passage **26**, preferably juxtaposed to the spray orifice **28** so as to be inserted into a nostril together. Preferably, LED **30** emits non-coherent light radiation having a narrow bandwidth centered at a wavelength suitable for rhinitis treatment, typically 660nm.

LED **30** is mounted in a support **32** formed of insulating material including two channels or bores which holds and separates the terminals **30a**, **30b** of the LED, as described above with regard to Figs. 3A, 3B and 3C. LED **30** is connected to a power source through an electric circuit, and the depressible flexible portion is arranged and configured to cause the electric circuit to close to activate the LED and also to actuate the spray dispensing mechanism, to provide both illumination and spray of liquid substance into the nozzle. Preferably, the LED illuminates the spray as well as the nostril.

A power source, here shown as a coin-type battery **34**, is seated within a battery compartment **36**. One LED terminal is disposed in battery compartment **36** under battery **34** and contacts the compatible pole of the battery **34**, while the second terminal **30a** is disposed above - but is flexibly kept a small distance away from - the upper side of the battery.

The cover-section **24** is placed over the intermediate-section **22**. It comprises a flexible upper wall **27** which can be used to actuate the spray apparatus. Upper wall **27** is sufficiently flexible to be depressed and engage a terminal of the LED so as to cause the terminal to contact the battery and light up the LED. Preferably, upper wall **27** can continue to press on the intermediate section **22** until it slides relative to base **20** and actuates the aerosol dispenser.

If desired, a push-button **40** can be provided protruding from the upper wall **27** for ease of actuation. A further protrusion **40a** (best seen in Figs. 4B and 4C) can be formed at the bottom side of button **40** disposed so that by pressing the button down, protrusion **40a** depresses LED terminal **30a** until it contacts the upper side of the battery **34**, closing the electric circuit which includes the battery and the LED, and lighting the LED. It will be appreciated that push-button **40** can be integrally formed with upper wall **27** of section **24** and/or protrusion **40a** can be integrally formed with upper wall **27**. Continued pressing of push-button **40** causes intermediate section **22** to slide relative to base **20**, causing the liquid dispensing mechanism to dispense liquid from the container.

It will be appreciated that the invention is not limited to what has been described hereinabove merely by way of example. Rather, the invention is limited solely by the claims which follow.

## Claims

1. A cap for a nasal spray apparatus (10), the cap including a spray dispensing mechanism and further comprising:
a base (20) sealingly mountable on a container (14) of liquid substance to be dispensed;
an intermediate section (22) arranged for reciprocating movement relative to the base (20), the intermediate section (22) including the spray dispensing mechanism and having a spray dispensing passage (26);
a cover-section (24) having a depressible flexible portion (27), coupled to the intermediate-section (22);
**characterized in that** it further comprises
a Light Emitting Diode (LED) (30) connected to a power source (34) through an electric circuit, the LED (30) mounted adjacent the spray dispensing passage (26) so as to be inserted into a nostril therewith;
and **in that**
t he depressible flexible portion (27) is arranged and configured to both cause closure of the electric circuit for activating the LED (30) and to actuate the spray dispensing mechanism, to provide both illumination and spray of liquid substance into the nostril.

2. The cap according to claim 1, wherein:
the LED (30) includes two terminals (30a, 30b);
the power source is a battery (34) seated in a battery compartment 36) disposed in the cap beneath the depressible flexible portion (27);
wherein one terminal of the LED (30) is disposed between the depressible flexible portion (27) and the battery (34) and not in contact with the battery, and the second terminal of the LED is disposed in the battery compartment (36) beneath and in contact with the battery (34).

3. The cap according to either claim 1 or claim 2, wherein the depressible flexible portion (27) includes a protruding push-button (40) on one side and an LED terminal engaging protrusion (40a) on the other side.

4. The cap according to claim 1, wherein the depressible flexible portion (27) includes a manual switch for closing the electric circuit to illuminate the LED (30).

## Patentansprüche

1. Eine Kappe für eine Nasensprayvorrichtung (10), wobei die Kappe einen Spray-Abgabemechanismus einschließt und weiter Folgendes umfasst:
eine Basis (20), die abdichtend an einem Behälter (14) für eine abzugebende flüssige Substanz montierbar ist;
einen intermediären Teil (22), angeordnet zum Hin- und Herbewegen relativ zur Basis (20), wobei der intermediäre Teil (22) den Spray-Abgabemechanismus einschließt und einen Spray-Abgabekanal (26) hat;
einen Deckelteil (24) mit einem komprimierbaren flexiblen Abschnitt (27),
gekoppelt mit dem intermediären Teil (22);
**dadurch gekennzeichnet, dass** sie weiter Folgendes umfasst:
eine Leuchtdiode (LED) (30), die über einen elektrischen Schaltkreis mit einer Stromquelle (34) verbunden ist, wobei die LED (30) neben dem Spray-Abgabekanal (26) montiert ist, um damit in ein Nasenloch eingeführt zu werden;
und dadurch, dass
der komprimierbare flexible Abschnitt (27) angeordnet und ausgebildet ist, um sowohl das Schließen des elektrischen Schaltkreis zur Aktivierung der LED (30) zu veranlassen als auch den Spray-Abgabemechanismus zu betätigen, um das Nasenloch sowohl mit Lichteinwirkung als auch mit einem Spray einer flüssigen Substanz zu versorgen.

2. Die Kappe gemäß Anspruch 1, wobei:
die LED (30) zwei Klemmen (30a, 30b) einschließt;
die Stromquelle eine Batterie (34) in einem Batteriefach (36) ist, angeordnet in der Kappe unterhalb des komprimierbaren flexiblen Abschnitts (27) ;
wobei eine Klemme der LED (30) zwischen dem komprimierbaren flexiblen Abschnitt (27) und der Batterie (34) angeordnet ist und nicht in Kontakt mit der Batterie steht und die zweite Klemme der LED in dem Batteriefach (36) unterhalb und in Kontakt mit der Batterie (34) angeordnet ist.

3. Die Kappe entweder gemäß Anspruch 1 oder gemäß Anspruch 2, wobei der komprimierbare flexible Abschnitt (27) einen vorstehenden Druckknopf (40) auf einer Seite und einen LED-Klemmen-Eingriffsvorsprung (40a) auf der anderen Seite einschließt.

4. Die Kappe gemäß Anspruch 1, wobei der komprimierbare flexible Abschnitt (27) einen manuellen Schalter zum Schließen des elektrischen Schaltkreises, um die LED (30) zu beleuchten, einschließt.

## Revendications

1. Embout pour pulvérisateur nasal (10), l'embout comportant un mécanisme de distribution par pulvérisation et comprenant en outre :
une base (20) pouvant être montée de façon glissante sur un récipient (14) d'une substance liquide à distribuer ;
une section intermédiaire (22) agencée pour suivre un mouvement de va-et-vient par rapport à la base (20), la section intermédiaire (22) comprenant le mécanisme de distribution par pulvérisation et comportant un passage de distribution par pulvérisation (26) ;
une section de capot (24) comportant une partie flexible pouvant être enfoncée (27), accouplée à la section intermédiaire (22) ;
**caractérisé en ce qu'**il comprend en outre :
une diode électroluminescente (DEL) (30) connectée à une source d'énergie (34) par l'intermédiaire d'un circuit électrique, la DEL (30) étant montée en position adjacente au passage de distribution par pulvérisation (26) afin d'être insérée dans une narine avec ce dernier ;
et **en ce que**
la partie flexible pouvant être enfoncée (27) est agencée et configurée à la fois pour provoquer la fermeture du circuit électrique pour activer la DEL (30) et pour actionner le mécanisme de distribution par pulvérisation, afin de fournir à la fois un éclairage et une pulvérisation de substance liquide dans la narine.

2. Embout selon la revendication 1, dans lequel :
la DEL (30) comprend deux bornes (30a, 30b) ;
la source d'énergie est une pile (34) logée dans un compartiment à pile (36) situé dans l'embout, sous la partie flexible pouvant être enfoncée (27) ;
dans lequel une borne de la DEL (30) est placée entre la partie flexible pouvant être enfoncée (27) et la pile (34) et non en contact avec la pile, et la deuxième borne de la DEL est placée dans le compartiment à pile (36), sous la pile (34) et en contact avec celle-ci.

3. Embout selon la revendication 1 ou 2, dans lequel la partie flexible pouvant être enfoncée (27) comprend un bouton-poussoir saillant (40) d'un côté et une protubérance de mise en prise de borne de DEL (40a) de l'autre côté.

4. Embout selon la revendication 1, dans lequel la partie flexible pouvant être enfoncée (27) comprend un interrupteur manuel pour fermer le circuit électrique afin d'allumer la DEL (30).
